# EUROPEAN PATENT SPECIFICATION

(11) **EP 2 790 683 B1**
(45) Date of publication and mention of the grant of the patent: **29.06.2016**
(21) Application number: 12834597.2
(22) Date of filing: 27.11.2012
(51) Int. Cl.: A61K 9/51, A61K 9/70

(54) **LIPID-BASED NANOCARRIER SYSTEMS FOR USING CANCER TREATMENT**
LIPID BASIERTER NANOTRÄGER FÜR DIE KREBSTHERAPIE
SYSTÈMES DE NANO-VÉHICULES À BASE DE LIPIDE DESTINÉ À ÊTRE UTILISÉS POUR LE TRAITEMENT CONTRE LE CANCER

(30) Priority: 28.11.2011 TR 201111743
(43) Date of publication of application: 22.10.2014
(73) Proprietor: Gürsoy, Reyhan Neslihan, Ankara (TR); Çevik, Özge, Ankara (TR)
(72) Inventor: Gürsoy, Reyhan Neslihan, Ankara (TR); Çevik, Özge, Ankara (TR)
(74) Representative: Bulut, Pinar
(86) International application number: PCT/TR2012/000203
(87) International publication number: WO 2013/100869

(56) References cited:
- CN-A- 102 240 265
- IE-A1- 20 050 639
- US-A1- 2009 048 322

## Description

### Technical Field

This system is about the formulation of a lipid-based drug delivery system designed for lymphatic targeting of a peptide which is specific to tumor lymphatics for the treatment of cancer.

### Backround of the Invention

The major factor that limits success in cancer therapy is the nonselectivity of the anticancer agents towards tumor cells and tissues. The main aim of cancer therapy is to destroy the cancer cell without affecting the normal tissues. Cancer cells carry specific receptors on the surface of their membranes. This enables targeting to these cells by molecular cell surface markers. At present, studies are being carried out utilizing specific monoclonal antibodies targeting special tumors. However, there are several factors that limit the efficient use of targeted antibody therapy. The high molecular size, low penetration and cellular uptake at the targeted region and nonspecific uptake by the liver and the RES are the main disadvantages of these systems. Thus, peptides have been considered as excellent alternatives to monoclonal antibodies as targeting agents to cancer.

Immunoglubulins and peptide ligands that bind to specific areas on blood/lymph vessels on certain tumors which have been isolated with phage display technique can be used for targeting cancer cells. In recent years, a nine amino acid, cyclic peptide, LyP-1 (CGNKRTRGC), which exhibited specificity to the tumor endothelium, has been isolated by the phage display technique utilizing MDA-MB-435 breast cancer xenografts. LyP-1 is not only a special marker for the tumor endothelial cells but also specific to the tumor lymphatic endothelia to which vascular peptides are not specific. It has been shown that following iv administration of LyP-1, the peptide accumulates heavily at the tumor and metastatic regions. In addition to localization and internalization in the tumor and endothelial cells, LyP-1 also induces apoptosis in the cells that it has been internalized. This renders LyP-1 invaluable among the other tumor-specific peptides. It has been demonstrated that following administration of LyP-1 to tumor-grown mice, this peptide inhibits tumor growth. The fact that LyP-1 plays a crucial role in targeting in cancer therapy brings out the possibility of the use of this peptide by conjugation to nanoparticular systems for the targeted cancer therapy.

### Drug Carrying Nanosystems

Nanotechnology contributes to develop cancer therapeutics and drugs that are used in neurological disorders. It is possible to develop nanosized drugs which can pass through biological barriers, such as blood brain barrier and tumor pores. Nanotechnological drugs make possible use of lower concentrations thus reduce the possible side effects of drugs because of their increased surface area and biological activity.

Self-Emulsifying Drug Delivery Systems (SEDDS) are nanosized lipid droplets which are composed of drugs, lipids, surface active agents or cosolvents/cosurfactants. The droplet size is 50 - 300 nm for SNEDDS (self-nanoemulsifying drug delivery systems) and under 50 nm for SMEDDS (self-microemulsifying drug delivery systems) following dilution with physiological fluids.

### Description of the Invention

This invention is about the formulation of LyP-1 peptide in nanocarrier systems, which is isolated from MDA MB-435 cells by phage display technique and has the amino acid sequence CGNKRTRGC.

The aim of this formulation is lymphatic targeting, and the oil/water partition coefficient should be higher than 5 for lymphatic transport.

In this system, LyP-1 by itself, or more active and stable peptide analogues, peptide derivatives, PEGylated forms or a conjugate with a chemotherapeutic, radiopharmaceutical agent, fatty acid and lipid can be entrapped in a SNEDDS, solid SEDDS, nanoemulsion, microemulsion, liposome, solid lipid nanoparticle, nanoparticle, nanocapsule, microparticle and preferably in SMEDDS formulations.

The formulation system in this invention as known as SMEDDS, composed oil, surfactant and cosolvent/surfactant, form oil droplets with an average size under 50 nm upon aqueous dilution in the gastrointestinal system.

It can be possible that the system passes through lymphatic circulation via M cells and enterocytes following the lipid absorption pathway, by filling the prepared lipid-based system into enteric coated capsules.

As a result of conjugation of the peptide with fatty acid derivatives, it is aimed to increase lipophilicity, facilitate entrance into cells and bind the targeted receptor after separation from the lipid.

For preparing the SMEDDS formulation, Span 20, 60, 65, 80, 85, Tween 20, 60, 80, 85, Tyloxapol, Gelucire 50/13, Labrafil M2130CS, Labrafil M1944CS, Labrafil M2125CS, Capryol 90, Capryol PGMC, Lauroglycol 90, Lauroglycol FCC, Plurol Oleique CC 497, Labrafac Lipophile WL 1349, Labrafac PG, Transcutol, Compritol HD5 ATO, Gelucire 43/01, Compritol 888 Pellets, Biogapress Vegetal BM297ATO, Compritol E ATO, Geloil SC, Transcutol V, Sodium Lauryl Sulphate, Cremophor EL, Triton X-100, Sodium Deoxycholate (Na-DOC) preferably Labrasol, Gelucire 44/14 or compositions can be used as a surfactant; Peceol, Maisine 35-1, soybean oil, corn oil, cotton oil, olive oil, sesame oil, castor oil, liquid paraffin, ethyl oleate and compositions can be used as oil phase and ethyl alcohol, isopropyl alcohol, poly ethylene glycol derivatives, glycerol, propylene glycol and mixtures can be used as a cosolvent.

Solid dosage forms like tablets, minitablets, soft capsules, enteric coated capsules, implants can be prepared by spray drying.

These systems can be administered by oral, intravenous, intratumoral, intramuscular, intravesical, intrathecal, intradermal, subcutaneous, transdermal, buccal, topical, transbuccal, transmucozal, sublingual, nasal, rectal, vaginal, intraocular, intramuscular, intralymphatic, intradermal, transpulmonary, inhalation, injection and implantation routes preferably for lymphatic targeting.

This system can be administered by the oral route directly in a dosage form like solution, emulsion, microemulsion, nanoemulsion, suspension, syrup, elixir, ointment or cream which contains suitable diluents like water and the other solvents, or in solid dosage forms like powder, tablet, gelatin capsule, minitablet, soft capsule, enteric coated capsule, suppository, patch, inhaler, spray, aerosol, solid SEDDS and spray dried pellets filled into hard gelatin capsules.

This system can be administered in emulsion, microemulsion or nanoparticular forms by intravenous injection.

This system can be used for diagnosis, imaging and treatment of osteosarcoma, breast and prostate cancers.

### Examples:

### Preparation and Characterization of Formulation

**Preformulation studies**: In preformulation studies, excipients were determined for drug carrying nanosystems. Oil, surfactant, cosolvent/surfactant combinations which gave technologically suitable microemulsion, nanoemulsion, SMEDDS formulations, were observed by construction of ternary phase diagrams. In preformulation studies, soybean oil, ethyl oleate, liquid paraffin, maisine and peceol were used as an oil phase, Triton X-100, Na-DOC, Tyloxapol, Cremophor, Labrasol and Gelucire 44/14 were used as surfactant and Glycerol, PEG 300, PEG 3000, Propylene Glycol were used as cosolvent. Table 1 shows the excipients used for the preparation of the formulations.

**Table 1: Excipients used for preparing suitable formulations**

| | **Surfactant Mixture** | **Oil phase** | **Cosolvent** |
|---|---|---|---|
| 1 | Triton X-100/Na-DOC (2:1) | Soybean oil | Glycerol |
| 2 | Cremophor/Na-DOC (2:1) | Soybean oil | Glycerol |
| 3 | Triton X-100/Na-DOC (2:1) | Soybean oil | PEG 300 |
| 4 | Triton X-100/Na-DOC (2:1) | Soybean oil | PEG 3000 |
| 5 | Triton X-100/Na-DOC (2:1) | Liquid paraffin | PEG 300 |
| 6 | Triton X-100/Na-DOC (2:1) | Ethyl oleate | PEG 300 |
| 7 | Tyloxapol/Na-DOC (2:1) | Liquid paraffin | PEG 300 |
| 8 | Tyloxapol/Na-DOC (2:1) | Ethyl oleate | PEG 300 |
| 9 | Tyloxapol/Na-DOC (4:1) | Liquid paraffin | PEG 300 |
| 10 | Tyloxapol/Na-DOC (3:1) | Liquid paraffin | PEG 300 |
| 11 | Labrasol/Na-DOC (4:1) | Liquid paraffin | PEG 300 |
| 12 | Gelucire 44/14/Na-DOC (4:1) | Liquid paraffin | PEG 300 |
| 13 | Labrasol/Tyloxapol (4:1) | Liquid paraffin | PEG 300 |
| 14 | Gelucire 44/14/Tyloxapol (4:1) | Liquid paraffin | PEG 300 |
| 15 | Labrasol/Tyloxapol (1:1) | Soybean oil | Propylene Glycol |
| 16 | Gelucire 44/14/Tyloxapol (1:1) | Soybean oil | Propylene Glycol |
| 17 | Labrasol/Gelucire (1:1) | Maisine | PEG 300 |
| 18 | Labrasol Gelucire (1:1) | Peceol | PEG 300 |

SMEDDS were prepared by mixing oil, surfactant and cosolvent homogenously on a 40 °C water bath. The mixture was then diluted with pH 7.4 phosphate buffer at a ratio of 1:10.

### Droplet Size and Zeta Potential Analysis

Formulations were analyzed in terms of droplet size and zeta potential utilizing a Malvern Zetasizer Nanoseries Nano ZS (UK) for two weeks (n=3).

### Morphological Analysis

The formulations were evaluated using polarized light microscopy (Leica DM EP, Germany) for the possibility of liquid crystal structures that give specific anisotropic images under polarized light.

### pH Analysis

pH of formulations was determined by a Sartorius PP-20 pH meter at certain time points for two weeks.

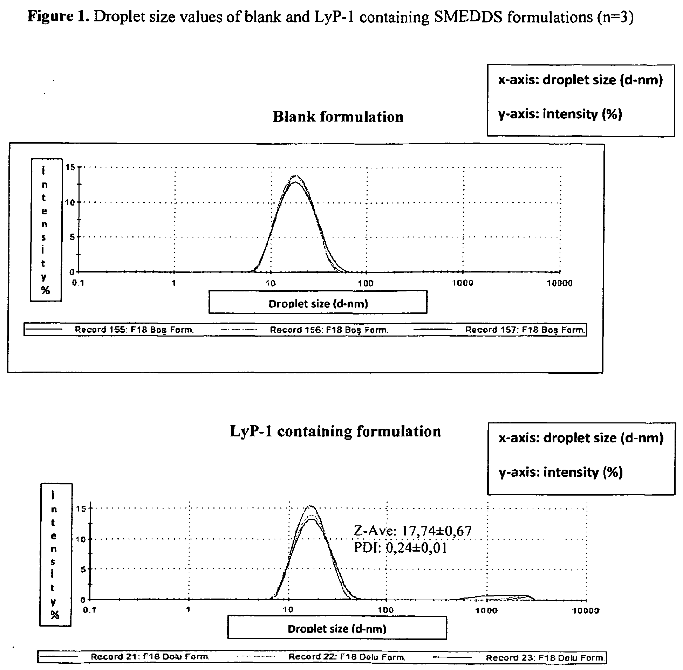

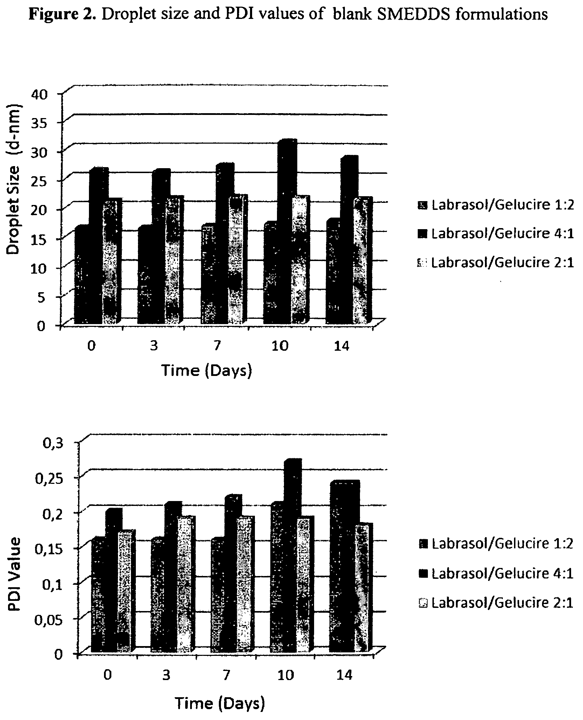

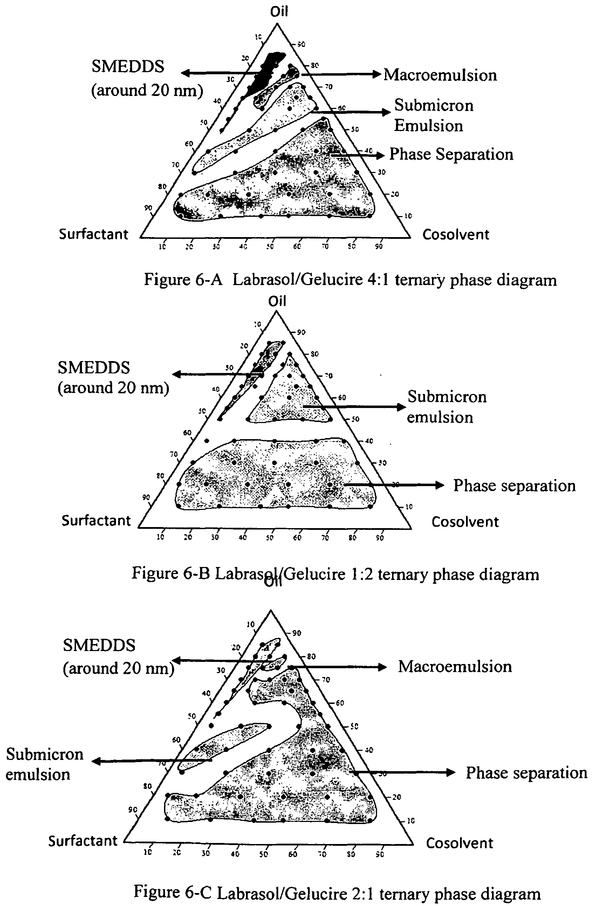

**Formulation studies:** Different surfactant ratios on ternary phase diagrams which gave stable formulations were determined. Suitable formulations were chosen and prepared with 1:1, 2:1, 4:1, 1:2 surfactant (Labrasol/Gelucire 44/14) ratios. These formulations were monitored for physical stability for 2 weeks. Three ternary phase diagrams were constructed with determined ratios of 2:1, 4:1, 1:2 and almost 50 formulations were prepared for each diagram. At the end of two week stability studies stable microemulsion regions were determined (Figure 6 A-C). The result of the stability studies are given in Table 2 and Figures 1. Droplet size and PDI values of blank SMEDDS formulations are given in Figure 2.

LyP-1-containing formulations were prepared with the suitable formulations. Morphological analysis, droplet size, zeta potential, and pH measurements were carried out within characterization studies.

**Table 2. Droplet size, PDI, zeta potential and pH values of blank SMEDDS (Ave ±SD)**

| Surfactant Ratio | Time (Day) | Droplet Size (d-nm) | PDI (Poly dispersity index) | Zeta Potential (mV) | pH |
|---|---|---|---|---|---|
| 4:1 n=10 | 0 | 26,38±4,10 | 0,20±0,04 | 0,57±2,51 | 7,16±0,05 |
| | 3 | 26,11±3,85 | 0,21±0,03 | -0,24±1,75 | 7,09±0,04 |
| | 7 | 27,17±4,69 | 0,22±0,04 | -1,13±1,62 | 7,00±0,05 |
| | 10 | 31,26±4,46 | 0,27±0,04 | -0,73±2,31 | 6,92±0,06 |
| | 14 | 28,48±3,67 | 0,24±3,67 | -1,77±1,85 | 6,83±0,06 |
| 1:2 n=10 | 0 | 16,53±0,38 | 0,16±0,02 | -0,98±2,54 | 7,28±0,06 |
| | 3 | 16,47±0,44 | 0,16±0,03 | -2,48±2,79 | 7,11±0,13 |
| | 7 | 16,74±1,44 | 0,16±0,04 | -1,90±1,94 | 7,01±0,12 |
| | 10 | 17,16±0,69 | 0,21±0.03 | -1,21±1,38 | 6,94±0,12 |
| | 14 | 17,76±0,93 | 0,24±0,04 | -1,34±2,36 | 6,85±0,11 |
| 2:1 n=6 | 0 | 21,17±0,46 | 0,17±0,03 | 0,71±3,98 | 7,20±0,05 |
| | 3 | 21,54±0,64 | 0,19±0,04 | -0,58±1,17 | 7,10±0,05 |
| | 7 | 21,73±0,74 | 0,19±0,03 | -2,16±1,6 | 6,96±0,06 |
| | 10 | 21,64±0,67 | 0,19±0,02 | -2,04±1,99 | 6,86±0,06 |
| | 14 | 21,49±0,71 | 0,18±0,01 | -3,18±2,03 | 6,75±0,07 |

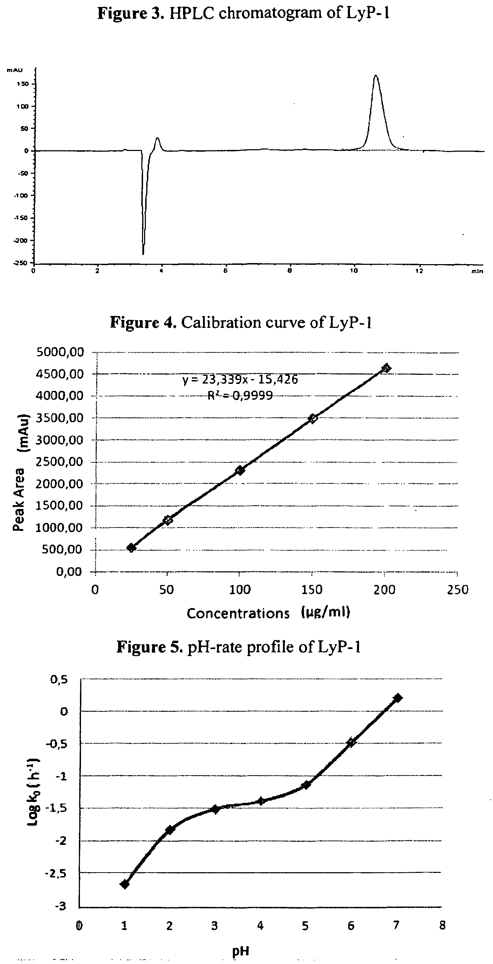

**Assay:** The wavelength of maximum absorbance for LyP-1 was determined using UV spectroscopy. An RP-HPLC method was developed and validated for quantitative analysis of LyP-1. In this method, RP C18 250x4,6 mm column, 5 % acetonitrile and 95 % aqueous solution with 0.1 % TFA were used as stationary phase and mobile phase, respectively. A typical chromatogram and the calibration curve and of LyP-1 can be seen in Figures 3 and 4, respectively.

**Stability Studies:** Stability studies of LyP-1 by itself and LyP-1-containing formulations were carried out as a function of temperature, pH and time. Newly developed HPLC method was used for quantitative analysis. The pH-rate profile of LyP-1 can be seen in Figure 5.

***In vitro* (cell culture) cytotoxicity studies:** Cytotoxicity of blank and peptide containing formulations were determined using Caco-2 cell line. MTT test was used to determine cytotoxic effects of formulations. PBS and SDS were used as positive and negative controls, respectively.

### Description of the Figures:

- Figure 1:: Droplet size values of blank and LyP-1 containing SMEDDS formulations (n=3)
- Figure 2:: Droplet size and PDI values of blank SMEDDS formulations
- Figure 3:: HPLC chromatogram of LyP-1
- Figure 4:: Calibration curve of LyP-1
- Figure 5:: pH-rate profile of LyP-1
- Figure 6-A:: Labrasol/Gelucire 4:1 ternary phase diagram
- Figure 6-B:: Labrasol/Gelucire 1:2 ternary phase diagram
- Figure 6-C:: Labrasol/Gelucire 2:1 ternary phase diagram

## Claims

1. A nanocarrier system suitable for cancer treatment or imaging comprising a self microemulsifying drug delivery system (SMEDDS) which is composed of surfactant, oil and cosolvent and containing LyP-1 peptide (CGNKRTRGC) by itself or as a conjugate or pegylated form.

2. A nanocarrier system according to claim 1, wherein LyP-1 peptide is a conjugate with a chemotherapeutic or radiopharmaceutical agent.

3. A nanocarrier system according to claim 1, wherein the droplet size is 15-50 nm.

4. A nanocarrier system according to claim 1, wherein it comprises Span 20, 60, 65, 80, 85, Tween 20, 60, 80, 85, Tyloxapol, Gelucire 50/13, Labrafil M2130CS, Labrafil M1944CS, Labrafil M2125CS, Capryol 90, Capryol PGMC, Lauroglycol 90, Lauroglycol FCC, Plurol Oleique CC 497, Labrafac Lipophile WL 1349, Labrafac PG, Transcutol, Compritol HD5 ATO, Gelucire 43/01, Compritol 888 Pellets, Biogapress Vegetal BM297ATO, Compritol E ATO, Geloil SC, Transcutol V, sodium lauryl sulfate, Cremophor EL, Triton X-100, sodium deoxycholate or mixtures of them as a surfactant.

5. Surfactant according to claim 4 is selected from Triton X-100, sodium deoxycholate, Tyloxapol, Cremophor, Labrasol, Gelucire 44/14 or mixtures of them, preferably Labrasol or Gelucire 44/14 or mixture of Labrasol and Gelucire 44/14.

6. A nanocarrier system according to claim 1, wherein it comprises Peceol, Maisine 35-1, soybean oil, corn oil, cottonseed oil, olive oil, sesame oil, castor oil, liquid paraffin, ethyl oleate or mixtures of them as oil phase.

7. Oil phase according to claim 6 is selected from soybean oil, ethyl oleate, liquid paraffin, Peceol, Maisine 35-1 or mixtures of them.

8. A nanocarrier system according to claim 1, wherein it comprises ethyl alcohol, isopropyl alcohol, polyethylene glycol derivatives, glycerol, propylene glycol or mixtures of them as cosolvent.

9. Cosolvent according to claim 8 is selected from glycerol, polyethylene glycol 300, polyethylene glycol 3000, propylene glycol or mixtures of them.

10. A nanocarrier system according to claim 1, wherein it comprises mixture of Labrasol and Gelucire 44/14 as the surfactant, Maisine or Peceol as the oil phase and polyethylene glycol 300 as the cosolvent.

## Patentansprüche

1. Ein Nanotransportsystem, geeignet für die Krebsbehandlung oder Abbilden eines selbst Mikroemulgator Arzneimittelabgabesystem (SMEDDS) umfasst, die aus Tensid, Öl und Colösungsmittel besteht und LyP-1 Peptid (CGNKRTRGC) alleine oder als Konjugate oder pegylierten Form enthält.

2. Nanotransportsystem nach Anspruch 1, wobei LyP-1-Peptid eineKonjugate mit chemotherapeutischem oder radiopharmazeutischem Mittel ist.

3. Nanotransportsystem nach Anspruch 1, wobei die Tröpfchengröße 15-50nm beträgt.

4. Nanotransportsystem nach Anspruch 1, wobei es eine Spanne 20, 60, 65, 80, 85, Tween 20, 60, 80, 85, Tyloxapol, Gelucire 50/13, Labrafil M2130CS, Labrafil M1944CS, Labrafil M2125CS, 90, Capryol PGMC, Lauroglycol 90, Lauroglycol FCC, Plurol Oleique CC 497, Labrafac Lipophile WL 1349, Labrafac PG, Transcutol, Compritol HD5 ATO, Gelucire 43/01, Compritol 888 Pellets, Biogapress Vegetal BM297ATO, Compritol E ATO, Geloil SC, Transcutol V, Natriumlaurylsulfat, Cremophor EL, Triton X-100, Natriumdesoxycholat oder Mischungen davon als Tensid umfasst.

5. Tensid nach Anspruch 4 wird aus Triton X-100, Natriumdesoxycholat ausgewählt, Tyloxapol, Cremophor, Labrasol, Gelucire 44/14 oder Mischungen davon, vorzugsweise Labrasol oder Gelucire 44/14 oder einer Mischung von Labrasol und Gelucire 44/14.

6. Nanotransportsystem nach Anspruch 1, wobei es Peceol, Maisine 35-1, Sojabohnenöl, Maisöl, Baumwollsaatöl, Olivenöl, Sesamöl, Rizinusöl, flüssiges Paraffin, Ethyloleat oder Gemischen davon als Ölphase enthält.

7. Ölphase nach Anspruch 6 wird aus Sojaöl, Ethyloleat ausgewählt, flüssiges Paraffin, Peceol, Maisine 35-1 oder Mischungen aus diesen.

8. Nanotransportsystem nach Anspruch 1, wobei es Ethylalkohol, Isopropylalkohol, Polyethylenglykolderivate, Glycerin, Propylenglykol oder Mischungen davon als Co-Solvens umfasst.

9. Cosolvens nach Anspruch 8 ist aus Glycerin, Polyethylenglykol 300, Polyethylenglycol 3000, Propylenglykol oder Mischungen davon ausgewählt.

10. Eine Nanotransportsystem nach Anspruch 1, wobei es Mischung aus Labrasol und Gelucire 44/14 als Tensid, Maisine oder Peceol als Ölphase und Polyethylenglykol 300 als Cosolvens umfasst.

## Revendications

1. Un système nano-porteur approprié pour le traitement ou l'imagerie du cancer comportant un système d'administration de médicaments auto-microémulsifiants (SDAMAM) composé de tensioactif, huile, et cosolvent et contenant le peptide deLyP-1 (CGNKRTRGC) en soi-même ou comme une forme conjuguée ou pégylée.

2. Un système nano-porteur selon la revendication 1, dans lequel le peptide de LyP-1 est conjugué avec un agent chimiothérapeutique ou radiopharmaceutique.

3. Un système nano-porteur selon la revendication 1, dans lequel la taille de gouttelette est 15-50 nm.

4. Un système nano-porteur selon la revendication 1, dans lequel on inclut Span 20, 60, 65, 80, 85, Tween 20, 60, 80, 85, Tyloxapol, Gelucire 50/13, Labrafil M2130CS, Labrafil M1944CS, Labrafil M2125CS, Capryol 90, CapryolPGMC, Lauroglycol 90, LauroglycolFCC, Plurol Oléique CC 497, Labrafac Lipophile WL 1349, LabrafacPG, Transcutol, Compritol HD5 ATO, Gelucire 43/01, Compritol 888 Pellets, Biogapress Végétal BM297ATO, Compritol E ATO, Geloil SC, Transcutol V, du lauryl sulfate de sodium, Cremophor EL, Triton X-100, du désoxycholate de sodium, ou leurs mélanges comme tensioactif.

5. Le tensioactif selon la revendication 4 est choisi parmi Triton X-100, le désoxycholate de sodium, Tyloxapol, Cremophor, Labrasol, Gelucire 44/14, ou leurs mélanges, de préférence, Labrasol ou Gelucire 44/14, ou le mélange de Labrasol et Gelucire 44/14.

6. Un système nano-porteur selon la revendication 1, dans lequel on inclut Paceol, Maisine 35-1, l'huile de soja, l'huile de maïs, l'huile de graine de coton, l'huile d'olive, l'huile de sésame, l'huile de ricin, la paraffine liquide, l'oléate d'éthyle, ou leurs mélanges comme phase huileuse.

7. La phase huileuse selon la revendication 6 est choisie parmi l'huile de soja, l'oléate d'éthyle, la paraffine liquide, Peceol, Maisine 35-1, ou leurs mélanges.

8. Un système nano-porteur selon la revendication 1, dans lequel on inclut de l'alcool éthylique, l'alcool isopropylique, des dérivés du glycol polyéthylenique, du glycérol, du propylène glycol, ou leurs mélanges comme tensioactif.

9. Le cosolvent selon la revendication 8 est choisi parmi le glycérol, le propylène glycol 300, le glycol polyéthylenique 3000, le propylène glycol, ou leurs mélanges.

10. Un système nano-porteur selon la revendication 1, dans lequel on inclut le mélange de Labrasol et Gelucire 44/14 comme tensioactif, Maisione ou Peceol comme la phase huileuse, et le glycol polyéthylenique 300 comme cosolvent.
